(19) Europäisches Patentamt
European Patent Office
Office européen des brevets

(11) Publication number : 0 153 580 B2

(12) NEW EUROPEAN PATENT SPECIFICATION

(45) Date of publication of the new patent specification : 17.03.93 Bulletin 93/11

(21) Application number : 85100569.4

(22) Date of filing : 21.01.85

(51) Int. Cl.[5] : C07D 471/04, A61K 31/44, A61K 31/505, // (C07D471/04, 221:00, 221:00), (C07D471/04, 239:00, 221:00)

# (54) Naphthyridine antibacterial compounds.

The file contains technical information submitted after the application was filed and not included in this specification

(30) Priority : 26.01.84 US 574120
26.01.84 US 574226

(43) Date of publication of application : 04.09.85 Bulletin 85/36

(45) Publication of the grant of the patent : 25.01.89 Bulletin 89/04

(45) Mention of the opposition decision : 17.03.93 Bulletin 93/11

(84) Designated Contracting States : AT BE CH DE FR GB IT LI LU NL SE

(56) References cited :
EP-A- 0 004 279

(56) References cited :
EP-A- 0 009 425
EP-A- 0 027 752
DE-A- 2 338 325
DE-A- 2 341 166
GB-A- 1 484 138
US-A- 4 284 629
US-A- 4 380 632
Programm and Abstracts of the Twenty-fourth Interscience Conference on Antimicrobial Agents and Chemotherapy, 8.-10. Oct.1984
J. Org. Chem. Vol.44(1979) p.310

(73) Proprietor : ABBOTT LABORATORIES
14th Street and Sheridan Road North St
North Chicago, Illinois 60064 (US)

(72) Inventor : Chu, Daniel Tim-Wo
204 Ashland Court
Vernon Hills Illinois (US)

(74) Representative : Modiano, Guido et al
c/o Modiano & Associati S.r.l. Via Meravigli, 16
I-20123 Milano (IT)

EP 0 153 580 B2

## Description

This invention relates to new naphthyridine derivatives having antibacterial properties, compositions containing the new naphthyridine derivatives and methods of treating mammalian patients with the new naphthyridine derivatives.

It is known that certain naphthyridine compounds exhibit antibacterial properties, notably certain 7-piperazinyl-4-oxo-1,8-naphthyridine-3-carboxylic acid.

In European Patent No. 9,425, there are disclosed certain 7-piperazinyl-6-fluoro-1,4-dihydro-4-oxo-1,8-naphthyridine-3-carboxylic acid derivatives which are substituted in the 1-position with an alkyl or vinyl substituent.

European Patent 27 752 discloses 7-pyrrolidinyl-6-fluoro-1,4-dihydro-4-oxo-1,8 naphthyridine-3-carboxylic acid derivatives substituted with ethyl in the 1-position.

This invention relates to novel antibacterial agents having the formula:

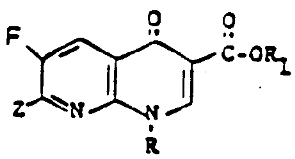

wherein $R_1$ is hydrogen or a carboxy protecting group; R is a phenyl group having the formula:

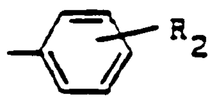

wherein $R_2$ is one or more substituents independently selected from the group consisting of hydrogen, halogen and hydroxy; and Z is a heterocyclic ring selected from 1-piperazinyl, 1-pyrrolidinyl and 1-piperidinyl and substituted derivatives thereof wherein the heterocyclic ring is substituted with one or more substituents independently selected from the group consisting of $C_1$ to $C_6$ alkyl, hydroxy, alkanoyl containing 1 to 6 carbon atoms, alkanoylamino containing 1 to 6 carbon atoms, an amine of the formula:

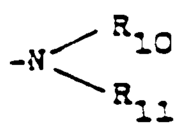

wherein $R_{10}$ and $R_{11}$ are each independently selected from the group consisting of hydrogen and $C_1$ to $C_6$ alkyl, provided that if Z is an amino substituted 1-pyrrolidinyl or an unsubstituted 1-piperazinyl R is only a substituted phenyl group, and pharmaceutically acceptable salts thereof.

A preferred group of compounds encompassed within the above general formula of the inventive compounds is that wherein Z is (1) mono-or di-substituted 1-pyrrolidinyl wherein the substituent is independently selected from $NH_2$ and $C_1$ to $C_3$ alkyl or (2) 1-piperazinyl or substituted 1-piperazinyl wherein the piperazinyl ring is substituted with one or more substituents independently selected from $C_1$ to $C_6$ alkyl, or $C_1$ to $C_6$ alkanoyl (the above proviso included); and pharmaceutically acceptable salts thereof.

As used herein, the term "halogen" refers to chloro, bromo, fluoro and iodo groups, while the term "$C_1$ to $C_6$ alkyl" refers to lower alkyl groups including methyl, ethyl, propyl, isopropyl, butyl, etc.

As used herein, the term "carboxy-protecting group" refers to and includes the residue of a carboxylic acid ester group. Such carboxy-protecting groups are well known to those skilled in the art, having been extensively used in the protection of carboxyl groups in the penicillin and cephalosporin fields, as described in U.S. Patent Nos. 3,840,556 and 3,719,667, the disclosures of which are incorporated herein by reference. In general, such carboxy-protecting groups can be relatively easily cleaved to yield the corresponding free carboxy group. Representative protecting groups include $C_1$ to $C_8$ alkyl (e.g., methyl, ethyl, tertiary butyl), benzyl and substituted derivatives thereof such as alkoxy and nitrobenzyl groups; also suitable are acyloxyalkyl groups such a pivaloyloxymethyl group.

The heterocyclic rings representing Z are, in accordance with the invention, aliphatic (saturated) heterocyclic rings containing 1 or 2 N atoms.

Also included are substituted derivatives of such heterocyclic rings wherein the substituent is one or more of a $C_1$ to $C_6$ alkyl group, hydroxy, alkanoyl containing 1-6 C atoms, alkanoylamino containing 1 to 6 C atoms, an amine group having the formula:

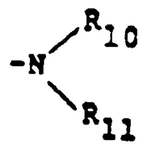

wherein $R_{10}$ and $R_{11}$ are each independently selected from the group consisting of hydrogen and $C_1$ to $C_6$ alkyl.

Also included within the scope of the present invention are pharmaceutically acceptable salts of the foregoing compounds. As used herein, the term "pharmaceutically acceptable salts" refers to non-toxic acid addition salts and alkaline earth metal salts of the compounds of formula I. The salts can be prepared in situ during the final isolation and purification of the compounds of formula I, or separately by reacting the free base or acid functions with a suitable organic acid or base. Representative acid addition salts include the hydrochloride, hydrobromide, sulphate, bisulphate, acetate, oxalate, valerate, oleate, palmitate, stearate, laurate, borate, benzoate, lactate, phosphate, tosylate, mesylate, citrate, maleate, fumarate, succinate, tartrate, glucoheptonate, lactobionate, lauryl sulfate salts and the like. Representative alkali or alkaline earth metal salts include the sodium, calcium, potassium and magnesium salts, etc. It has been found that the compounds of the present invention possess antibacterial activity against a wide spectrum of gram positive and gram negative bacteria, as well as enterobacteria. The compounds of the invention are therefor useful in the antibiotic treatment of susceptible bacterial infections in both humans and animals. In addition, the compounds, by reason of their in vitro activity, may be used in scrub solutions for surface inhibition of bacterial growth.

Susceptible organisms generally include those gram positive and gram negative, aerobic and anaerobic organisms whose growth can be inhibited by the compounds of the invention such as Staphylococcus, Lactobacillus, Streptococcus, Sarcina, Escherichia, Enterobacter, Klebsiella, Pseudomonas, Acinetobacter, Proteus, Citrobacter, Nisseria, Baccillus, Bacteroides, Peptococcus, Clostridium, Salmonella, Shigella, Serratia, Haemophilus, Brucella, and other organisms. In addition to exhibiting highly effective antibacterial activity, the compounds of the invention exhibit increased and improved solubility characteristics as compared with prior naphthyridine-3-carboxylic acid compounds in the art.

The compounds of Formula I may also be formulated into compositions together with pharmaceutically acceptable carriers for parenteral injection, for oral administration in solid or liquid form, for rectal administration, and the like.

Compositions according to the invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying, and dispersing agents. They may be sterilized, for example, by filtration through a bacteria-retaining filter, or by incorporating sterilizing agents into the compositions. They can also be manufactured in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert diluent such as sucrose, lactose or starch. Such dosage forms can also comprise, as is normal practice, additional substances other than diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixers containing inert diluents commonly used in the art, such as water. Besides such inert diluents, compositions can also include adjuvants, such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the compositions of the invention may be varied so as to obtain an amount of active ingredient effective to achieve antibacterial activity in accordance with the desired method

of administration The selected dosage level therefore depends upon the nature of the active compound administered, the route of administration, the desired duration of treatment and other factors. Generally, daily dosage levels of the compounds of formula I of about 0.1 to about 750, more preferably about 0.25 to about 500 ant most preferably about 0.5 to about 300 mg. of active ingredient per kg. of body weight are effective when administered orally to a mammalian patient suffering from an infection caused by a susceptible organism. If desired, the daily dose may be divided into multiple doses for administration, e.g., two to four times per day.

The foregoing may be better understood from the following examples, which are presented for purposes of illustration and are not intended to limit the scope of the inventive concepts.

A general outline is also given in the following Preparation of the method usable for preparing the compounds according to the present invention.

Preparation

1-Phenyl-6-fluoro-1,4-dihydro-4-oxo-7-(1-piperazinyl)-1,8-naphthyridine-3-carboxylic acid

(a) To a solution of 1.g of ethyl-2,6-dichloro-5-fluoronicotinate in 5 ml. of trifluoroacetic acid, 6 ml. 6 N HCl is added. The solution is heated for 16 hours and cooled and extracted with a methylene chloride. The methylene chloride solution is extracted with saturated sodium bicarbonate solution. The aqueous extract is acidified to pH 3 and then extracted with methylene chloride. After drying, it yields 0.767 g. 2,6-dichloro-5-fluoronicotinic acid. This compound (0.5 g.) is then suspended in 10 ml. benzene and 1.7 ml. of thionyl chloride and 2 drops of dimethylformamide. After refluxing for 30 minutes, the reaction mixture is evaporated to dryness to give the corresponding acid chloride. This is added to a solution of 2.54 g. of ethyl malonate monoester in 20 ml. of THF solution containing 0.52 g. of n-butyl lithium at -65°C. The solution is allowed to warm up to room temperature, and then acidified and extracted with ether. The ether extract is washed with saturated $NaHCO_3$ and then water, and dried to yield 0.49 g. ethyl 2,6-dichloro-5-fluoronicotinylacetate.

(b) A solution of 3.8 g. of the ethyl 2,6-dichloro-5-fluoro-nicotinylacetate beta-ketoester of 1(a) in 3.5 ml. of triethylorthoformate and 10 ml. of acetic anhydride is heated at 135°C for 1-1/2 hours with the removal of the ethyl acetate formed during the reaction. The solution is evaporated under reduced pressure to a mobile oil. The oil is then dissolved in 150 ml. of methylene chloride and 1.5 ml. of aniline is added into the solution. After 1 hour, the solution is evaporated to dryness and purified through silica gel column yielding 3.7 g. ethyl 3-anilino-2-(2,6-dichloro-5-fluoro-nicotinyl)acrylate.

(c) To a cold solution of 3.5 g. of the preceding product in 100 ml. tetrahydrofuran is slowly added 240 mg. of a 60% sodium hydride-in-oil suspension. It is then heated for 1 hour and cooled, and 1 liter of water is added. The mixture is then filtered and the solid is washed with a 1:1 hexane/ether solution to obtain 2.1 g. ethyl 1-phenyl-6-fluoro-7-chloro-1,4-dihydro-4-oxo-1,8-naphthyridine-3-carboxylate.

(d) To a suspension of 5.2 g. of the preceding product in 30 ml. THF is added a sodium hydroxide solution (0.74 g. in 20 ml. $H_2O$). The mixture is heated at 80°C for 1 hour resulting in a clear solution which is evaporated under reduced pressure to dryness. The solid is dissolved in 200 ml. $H_2O$, and 2 ml. acetic acid is added. The resulting precipitate is filtered and washed with cold water, crystallized to produce 1-phenyl-6-fluoro-7-chloro-1,4-dihydro-4-oxo-1,8-naphthyridine-3-carboxylic acid.

(e) To a solution of 2.0 g. of 1-phenyl-6-fluoro-7-chloro-1,4-dihydro-4-oxo-1,8-naphthyridine-3-carboxylic acid in 20 ml. of 1-methyl-2-pyrrolidinone at 65°C is added 2 ml. piperazine. After stirring at 65°C for 20 hours, the solvent is removed by reduced pressure to dryness. Ethanol is added to the residue and the resulting mixture is filtered and washed with ether and then washed with very small amounts of cold water to give a solid. The resulting dried solid is suspended in 30 ml. $H_2O$ and 5 ml. IN HCl is added to and warmed to dissolve. Removal of the solvent under reduced pressure gives hydrochloride salt of 1-phenyl-6-fluoro-1,4-dihydro-4-oxo-7-(1-piperazinyl)-1,8-naphthyridine-3 carboxylic acid.

To the hydrochloride salt is added one molar equivalent of an aqueous solution of sodium hydroxide, and the resulting precipitate is filtered to obtain 1-phenyl-6-fluoro-1,4-dihydro-4-oxo-7-(1-piperazinyl)-1,8-naphthyridine-3-carboxylic acid.

(f) Alternately, the title compound is prepared as follows: To a solution of 1.6 g. of ethyl 1-phenyl-6-fluoro-7-chloro-1,4-dihydro-4-oxo-1,8-naphthyridine-3 carboxylate, the product of 1(c) in 50 ml. of methylene chloride is added 1 ml. of triethylamine and 710 mg. of acetylpiperazine. After heating for 2 hours, the solvent is washed with 50 ml. of 1 N HCl solution and then with water. The methylene chloride solution is dried and evaporated to dryness, yielding 2.1 g. of ethyl 1-phenyl-6-fluoro-7-(4-acetyl-1-piperazinyl)-1,4-dihydro-4-oxo-1,8-naphthyridine-3-carboxylate.

A suspension of 2.1 g. of the preceding compound in 25 ml. of 3 N HCl solution is heated at 80°C for 6

hours. The solvent is removed giving the hydrochloride salt of the title compound. This solid is dissolved in 100 ml. water. The pH of the solution is adjusted to pH 7 by the addition of 10% sodium hydroxide. The precipitate is filtered and washed with cold water yielding 1.8 g. of 1-phenyl-6-fluoro-1,4-dihydro-4-oxo-7-(1-piperazinyl)-1,8-naphthyridine-3-carboxylic acid.

Example 1

1-phenyl-6-fluoro-1,4-dihydro-4-oxo-7-(1-(4-methyl)piperazinyl)-1,8-naphthyridine-3-carboxylic acid

The procedure of the Preparation can be repeated, replacing piperazine in step (e) with N-methyl-piperazine to obtain 1-phenyl-6-fluoro-1,4-dihydro-4-oxo-7-(1-(4-methyl)piperazinyl)-1,8-naphthyridine3-carboxylic acid and its hydrochloride salt.

Example 2

1-phenyl-6-fluoro-1,4-dihydro-4-oxo-7 (1-pyrrolidinyl)-1,8-naphthyridine-3-carboxylic acid

In the described fashion of the Preparation replacing piperazine in step (e) with pyrrolidine, one can obtain 1-phenyl-6-fluoro-1,4-dihydro-1-oxo-7(1-pyrrolidinyl)-1,8-naphthyridine-3-carboxylic acid in good yield.

Example 3

1-phenyl-6-fluoro-1,4-dihydro-4-oxo-7-(3-hydroxy-1-pyrrolidinyl)-1,8-naphthyridine-3-carboxylic acid

The procedure of the Preparation can be repeated replacing piperazine in step (e) with 3-hydroxy-pyrrolidine to obtain 1-phenyl-6-fluoro-1,4-dihydro-4-oxo-7-(3-hydroxy-1-pyrrolidinyl)-1,8-naphthyridine-3-carboxylic acid.

Example 4

1-phenyl-6-fluoro-1,4-dihydro-4-oxo-7-(1-piperidinyl)-1,8-naphthyridine-3-carboxylic acid

The procedure of the Preparation can be repeated replacing piperazine in step (e) with piperidine to obtain 1-phenyl-6-fluoro-1,4-dihydro-4-oxo-7-(1-piperidinyl)-1,8-naphthyridine-3-carboxylic acid.

Example 5

1-phenyl-6-fluoro-1,4-dihydro-4-oxo-7-(3,5-dimethyl-1-pirerazinyl)-1,8-naphthyridine-3-carboxylic acid

In the described fashion as the Preparation replacing piperazine in step (e) with 2,6-dimethyl piperazine, one can obtain 1-phenyl-6-fluoro-1,4-dihydro-4-oxo-7-(3,5-dimethyl-1-piperazinyl)-1,8-naphthylidine-3-carboxylic acid and its hydrochloride salt.

Example 6

1-p-fluorophenyl-6,fluoro-1,4-dihydro-4-oxo-7-(1-piperazinyl)-1,8-naphthyridine-3-carboxylic acid

(a) In the described fashion as in Preparation (b) replacing aniline with p-fluororaniline, one can obtain the ethyl 3-(4-fluoroanilino)-2-(2,6-dichloro-5-fluoro-nicotinyl)acrylate.
(b) By following the Preparation (c) and (d), the preceding compound(s) can yield 7-chloro-1-p-fluorophenyl-6-fluoro-1,4-dihydro-4-oxo-1,8-naphthyridine-3-carboxylic acid.
(c) In the described fashion as Preparation (e) the above acid can give the desired 1-p-fluorophenyl-6-fluoro-1,4-dihydro-4-oxo-7-(1-piperazinyl)-1,8-naphthyridine-3-carboxylic acid and its hydrochloride salt.

Example 7

In the described fashion as Preparation (e), replacing the acid with the acid of the product of Example 9 (b) and also replacing piperazine with an appropriate amine such as N-methylpiperazine, pyrrolidine, 3-hydroxypyrrolidine, 3-acetamidopyrrolidine, piperidine, morpholine, thiomorpholine, 2,6-dimethyl piperazine, homopiperazine, diethylamine and 2,2-dimethylhydrazine, one can obtain the following compounds:

(a) 1-p-fluorophenyl-6,fluoro-1,4-dihydro-4-oxo-7-(1-(4-methyl)piperazinyl)-1,8-naphthyridine-3-carboxylic acid.

(b) 1-p-fluorophenyl-6-fluoro-1,4-dihydro-4-oxo-7-(1-pyrrolidinyl)-1,8-naphthyridine-3-carboxylic acid.

(c) 1-p-fluorophenyl-6-fluoro-1,4-dihydro-4-oxo-7-(3-hydroxy-1-pyrrolidinyl)-1,8-naphthyridine-3-carboxylic acid.

(d) 1-p-fluorophenyl-6-fluoro-1,4-dihydro-4-oxo-7-(3-acetamino-1-pyrrolidinyl)-1,8-naphthyridine-3-carboxylic acid.

(e) 1-p-fluorophenyl-6-fluoro-1,4-dihydro-4-oxo-7-(1-piperidinyl)-1,8-naphthyridine-3-carboxylic acid.

(f) 1-p-fluorophenyl-6-fluoro-1,4-dihydro-4-oxo-7-(3,5-dimethyl-1-piperazinyl)-1,8-naphthyridine-3-carboxylic acid.

## Example 8

### 1-p-fluorophenyl-6-fluoro-1,4-dihydro-4-oxo-7-(3-amino-1-pyrrolidinyl)-1,8-naphthyridine-3-carboxylic acid

The compound of Example 7 (d) can be hydrolised with hydrochloric acid at 80°C to give 1-p-fluorophenyl-6-fluoro-1,4-dihydro-4-oxo-7-(3-amino-1-pyrrolidinyl)-1,8-naphthyridine-3-carboxylic acid.

## Example 9

### 1-p-hydroxyphenyl-6-fluoro-1,4-dihydro-4-oxo-7-(1-piperazinyl)-1,8-naphthyridine-3-carboxylic acid

(a) In the described fashion as Preparation (b) replacing aniline with p-hydroxyaniline, one can obtain the ethyl 3-(4-hydroxyanilino)-2-(2,6-dichloro-5-fluoro-nicotinyl)acrylate.

(b) By following the Preparation (c) and (d), the preceding compound can yield 7-chloro-1-p-hydroxyphenyl-6-fluoro-1,4-dihydro-4-oxo-1,8-naphthyridine-3-carboxylic acid.

(c) In the described fashion as Preparation (e), the above acid can give the desired 1-p-hydroxyphenyl-6-fluoro-1,4-dihydro-4-oxo-7-(1-piperazinyl)-1,8-naphthyridine-3-carboxylic acid and its hydrochloride salt.

## Example 10

In the described fashion as Preparation (e), replacing the acid with the acid of the product of Example 9(b) and also replacing piperazine with an appropriate amine such as N-methylpiperazine, pyrrolidine, 3-hydroxy-pyrrolidine, 3-acetamidopyrrolidine, piperidine, morpholine, thiomorpholine, 2,6-dimethylpiperazine, homopiperazine, diethylamine and 2,2-dimethylhydrazine, one can obtain the following compounds:

(a) 1-p-hydroxyphenyl-6-fluoro-1,4-dihydro-4-oxo-7-(4-methyl)piperazinyl)-1,8-naphthyridine-3-carboxylic acid and its hydrochloride salt.

(b) 1-p-hydroxyphenyl-6-fluoro-1,4-dihydro-4-oxo-7-(1-pyrrolidinyl)-1,8-naphthyridine-3-carboxylic acid.

(c) 1-p-hydroxyphenyl-6-fluoro-1,4-dihydro-4-oxo-7-(3-hydroxyl-1-pyrrolidinyl)-1,8-naphthyridine-3-carboxylic acid.

(d) 1-p-hydroxyphenyl-6-fluoro-1,4-dihydro-4-oxo-7-(3-acetamino-1-pyrrolidinyl)-1,8-naphthyridine-3-carboxylic acid.

(e) 1-p-hydroxyphenyl-6-fluoro-1,4-dihydro-4-oxo-7-(1-piperidinyl)-1,8-naphthyridine-3-carboxylic acid.

(f) 1-p-hydroxyphenyl-6-fluoro-1,4-dihydro-4-oxo-7-(3,5-dimethyl-1-piperazinyl)-1,8-naphthyridine - 3 - carboxylic acid.

## Example 11

### 1-p-hydroxyphenyl-6-fluoro-1,4-dihydro-4-oxo-7-(3-amino-1-pyrrolidinyl)-1,8-naphthyridine-3-carboxylic acid

In the described fashion of 8, the compound of Example 10 (d) gave 1-p-hydroxyphenyl-6-fluoro-1,4-dihydro-4-oxo-7-(3-amino-1-pyrrolidinyl)-1,8-naphthyridine-3-carboxylic acid.

Example 12

In the described fashion as Preparation (a-d), replacing aniline with an appropriate amine (R-$NH_2$), one can obtain the additional 1-substituted, 7-chloro-6-fluoro-1,4-dihydro-4-oxo-1,8-naphthyridine-3-carboxylic acid as listed in Table I.

Table I

| Aniline replacement ($RNH_2$) | R- Substituent |
|---|---|
| a) o-fluoroaniline | o-fluorophenyl |
| b) p-chloroaniline | p-chlorophenyl |
| c) o-chloroaniline | o-chlorophenyl |
| d) 2,4-difluoroaniline | 2,4-difluorophenyl |
| e) 2-hydroxyaniline | 2-hydroxyphenyl |
| f) 2-hydroxy-4-fluoroaniline | 2-hydroxy-4-fluorophenyl |
| g) 2,4-dihydroxyaniline | 2,4-dihydroxyphenyl |
| h) 3-chloro-4-hydroxyaniline | 3-chloro-4-hydroxyphenyl |

Example 13

In the described fashion of Preparation (e), replacing the acid with the acid of the compounds listed in Table I of Example 12 and also replacing piperazine with an appropriate amine such as N-methylpiperazine, N-acyl piperazine, pyrrolidine, 3-hydroxypyrrolidine, 3-acetamido-pyrrolidine, 3-dimethylaminopyrrolidine, piperidine, morpholine, thiomorpholine, 2,6-dimethylpiperazine, homo-piperazine, dimethylamine and 2,2-dimethylhydrazine, one can obtain the following additional compounds as summarized in Table II.

**Table II**

| | Piperazine Replacement ZH | R- Substituent of Example 15 | Compound I Obtained ($R_1$=H) R | Z |
|---|---|---|---|---|
| 1. | piperazine | o-fluorophenyl | o-fluorophenyl | piperazinyl |
| 2. | N-methylpiperazine | 2,4-difluorophenyl | 2,4-difluoro-phenyl | N-methylpiper-azinyl |
| 3. | N-methylpiperazine | o-fluorophenyl | o-fluorophenyl | N-methylpiper-azinyl |
| 4. | N-methylpiperazine | o-hydroxyphenyl | o-hydroxyphenyl | N-methylpiper-azinyl |
| 5. | pyrrolidine | o-fluorophenyl | o-fluorophenyl | pyrrolidinyl |
| 6. | 3-hydroxypyrro-lidine | o-fluorophenyl | o-fluorophenyl | 3-hydroxy-pyrrolidinyl |
| 7. | 3-hydroxypyrro-lidine | p-chlorophenyl | p-chlorophenyl | 3-hydroxy-pyrrolidinyl |
| 8. | 3-acetamido-pyrrolidine | o-fluorophenyl | o-fluorophenyl | 3-acetamido-pyrrolidinyl |

| | | | |
|---|---|---|---|
| 9. 3-dimethylamino-pyrrolidine | p-fluorophenyl | p-fluorophenyl | 3-dimethylamino-pyrrolidinyl |
| 10. piperidine | o-fluorophenyl | o-fluorophenyl | piperadinyl |
| 11. 2,6-dimethyl-piperazine | o-fluorophenyl | o-fluorophenyl | 3,5-dimethyl-piperazinyl |

| | | | |
|---|---|---|---|
| 12. piperazine | 3-chloro-4-hydroxyphenyl | 3-chloro-4-hydroxyphenyl | piperazinyl |
| 13. N-methyl-piperazine | 3-chloro-4-hydroxyphenyl | 3-chloro-4-hydroxyphenyl | 4-methyl-piperazinyl |
| 14. 4-acetyl-piperazine | p-fluorophenyl | p-fluorophenyl | 4-acetyl-piperazinyl |
| 15. 4-propionyl-piperazine | p-fluorophenyl | p-fluorophenyl | 4-propionyl-piperazinyl |
| 16. piperazine | 3-fluoro-4-hydroxy-phenyl | 3-fluoro-4-hydroxyphenyl | piperazinyl |

## Claims

### Claims for the following Contracting States : BE, CH, DE, FR, GB, IT, LI, LU, NL, SE

**1.** A compound having the formula:

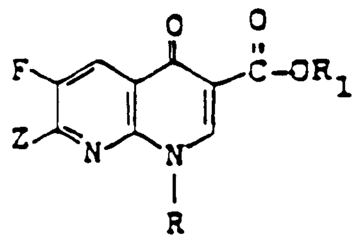

wherein $R_1$ is hydrogen or a carboxy protecting group; R is a phenyl group having the formula:

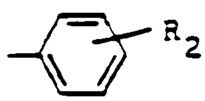

wherein $R_2$ is one or more substituents independently selected from the group consisting of hydrogen, halogen and hydroxy; and Z is a heterocyclic ring selected from 1-piperazinyl, 1-pyrrolidinyl and 1-piperidinyl and substituted derivatives thereof wherein the heterocyclic ring is substituted with one or more substituents independently selected from the group consisting of $C_1$ to $C_6$ alkyl, hydroxy, alkanoyl containing 1 to 6 carbon atoms, alkanoylamino containing 1 to 6 carbon atoms, an amine of the formula:

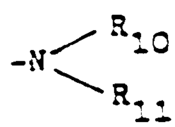

wherein $R_{10}$ and $R_{11}$ are each independently selected from the group consisting of hydrogen and $C_1$ to $C_6$ alkyl, provided that if Z is an amino substituted 1-pyrrolidinyl or an unsubstituted 1-piperazinyl, R is only a substituted phenyl group, and pharmaceutically acceptable salts thereof.

**2.** A compound as defined in claim 1 wherein R is p-hydroxyphenyl, Z is 1-piperazinyl, and $R_1$ is hydrogen.

**3.** A compound as defined in claim 1 wherein R is p-fluorophenyl, Z is 4-methyl-1-piperazinyl or 1-piperazinyl and $R_1$ is hydrogen.

**4.** A compound as defined in claim 1 wherein R is p-fluorophenyl or o,p-difluorophenyl, $R_1$ is hydrogen and Z is 3-amino-1-pyrrolidinyl.

**5.** A composition having antibacterial activity in pharmaceutical dosage form containing a diluent and a compound as defined in claim 1.

**6.** Use of a compound as defined in claim 1 for preparing a drug for treating a bacterial infection in a patient.

### Claims for the following Contracting State : AT

**1.** A process for preparing a compound having the formula:

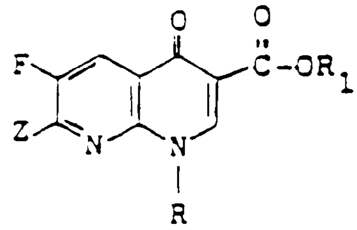

wherein $R_1$ is hydrogen or a carboxy protecting group; R is a phenyl group having the formula:

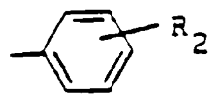

wherein $R_2$ is one or more substituents independently selected from the group consisting of hydrogen, halogen and hydroxy, and Z is a heterocyclic ring selected from 1-piperazinyl, 1-pyrrolidinyl and 1-piperidinyl and substituted derivatives thereof wherein the heterocyclic ring is substituted with one or more substituents independently selected from the group consisting of $C_1$ to $C_6$ alkyl, hydroxy, alkanoyl containing 1 to 6 carbon atoms, alkanoylamino containing 1 to 6 carbon atoms, an amine of the formula:

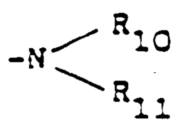

wherein $R_{10}$ and $R_{11}$ are each independently selected from the group consisting of hydrogen and $C_1$ to $C_6$ alkyl, provided that if Z is an amino substituted 1-pyrrolidinyl or an unsubstituted 1-piperazinyl, R is only a substituted phenyl group; and pharmaceutically acceptable salts thereof, comprising:
(i) reacting ZH with a 7-halo-1-R-substituted-6-fluoro-1,4-dihydro-4-oxo-naphthyridine-3-carboxylic acid (A) or ester thereof, wherein Z, R and $R_1$ are as defined above and X is a halogen

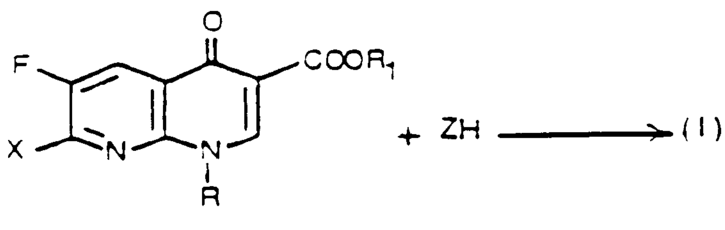

(A)

to obtain the desired compound (I).

2. A process according to claim 1 further comprising one or more of the following preliminary steps:
(a) reacting a 2,6-dihalo-5-fluoronicotinylhalide with a malonate monoester to give a 2,6-dihalo-5-fluoro-nicotinylacetate (B):

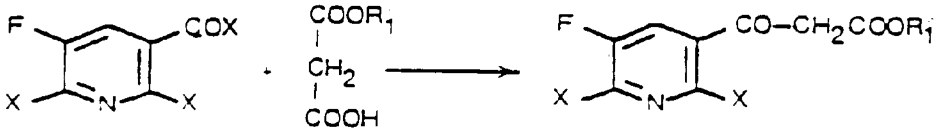

(B)

wherein $R_1$ is a carboxy protecting group,

(b) reacting the beta-ketoester (B) with triethylorthoformate and then with an aniline $RNH_2$ to give a 3-anilino-2-(2,6-dihalo-5-fluoronicotinyl) acrylate (C):

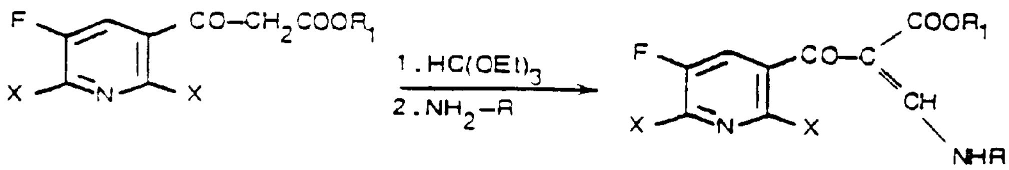


(C)

wherein R has the meaning as defined above,

(c) cyclizing compound (C) to obtain the 1-phenyl-6-fluoro-7-halo-1,4-dihydro-4-oxo-1,8-naphthyridine-3-carboxylate (A):

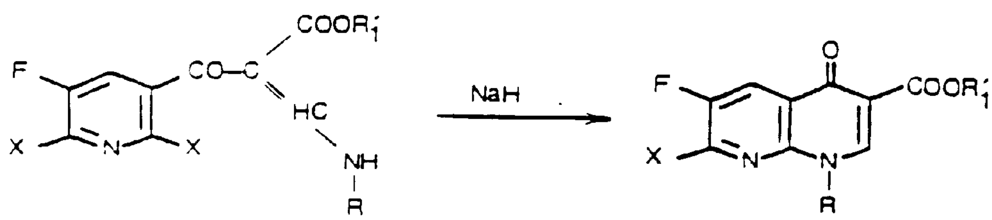


(A

(d) and optionally hydrolyzing (A) to obtain the corresponding 3-carboxylic acid form thereof.

3. A process as defined in claim 1 wherein R is p-hydroxyphenyl, Z is 1-piperazinyl, and $R_1$ is hydrogen.

4. A process as defined in claim 1 wherein R is p-fluorophenyl, Z is 4-methyl-1-piperazinyl or 1-piperazinyl and $R_1$ is hydrogen.

5. A process as defined in claim 1 wherein R is p-fluorophenyl or o,p-difluorophenyl, $R_1$ is hydrogen and Z is 3-amino-1-pyrrolidinyl.

6. A process for preparing a composition having antibacterial activity comprising mixing a compound prepared by a process according to claim 1 with a diluent.

**Patentansprüche**

**Patentansprüche für folgende Vertragsstaaten : BE, CH, DE, FR, GB, IT, LI, LU, NL, SE**

1. Eine Verbindung der Formel:

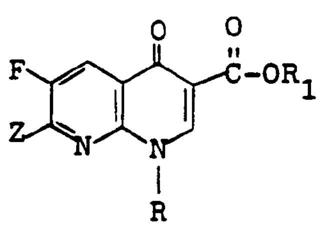

,

worin $R_1$ Wasserstoff oder eine Carboxyschutzgruppe ist; R eine Phenylgruppe der Formel:

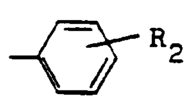

ist, wobei $R_2$ einen oder mehrere Substituenten bedeutet, die unabhängig voneinander aus der Gruppe ausgewählt sind, die aus Wasserstoff, Halogen und Hydroxy besteht; und Z ein aus 1-Piperazinyl, 1-Pyrrolidinyl and 1-Piperidinyl und substituierten Derivaten davon ausgewählter heterocyclischer Ring ist, in welchen der heterocyclische Ring durch einen oder mehrere Substituenten substituiert ist, die unabhängig voneinander aus der Gruppe ausgewählt sind, welche aus $C_1$-$C_6$-Alkyl, Hydroxy, Alkanoyl mit 1 bis 6 Kohlenstoffatomen, Alkanoylamino mit 1 bis 6 Kohlenstoffatomen, und einem Amin der Formel:

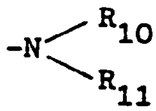

besteht, wobei $R_{10}$ und $R_{11}$ jeweils unabhängig voneinander aus der Gruppe ausgewählt sind, die aus Wasserstoff und $C_1$-$C_6$-Alkyl besteht, mit der Maßgabe, daß, falls Z einen Amino-substituiertes 1-Pyrrolidinyl oder ein unsubstituiertes 1-Piperazinyl bedeutet, R nur eine substituierte Phenylgruppe ist; und pharmazeutisch annehmbare Salze davon.

2. Eine Verbindung wie in Anspruch 1 definiert, worin R p-Hydroxyphenyl ist Z 1-Piperazinyl ist, und $R_1$ Wasserstoff ist.

3. Eine Verbindung wie in Anspruch 1 definiert, worin R p-Fluorphenyl ist, Z 4-Methyl-1-Piperazinyl oder 1-Piperazinyl ist, und $R_1$ Wasserstoff ist.

4. Eine Verbindung wie in Anspruch 1 definiert, worin R p-Fluorphenyl oder o,p-Difluorphenyl ist, $R_1$ Wasserstoff ist, und Z 3-Amino-1-pyrrolidinyl ist.

5. Eine antibakterielle Wirksamkeit aufweisende Zusammensetzung in pharmazeutischer Dosierungsform, enthaltend ein Verdünnungsmittel und eine Verbindung wie in Anspruch 1 definiert.

6. Die Verwendung einer Verbindung wie in Anspruch 1 definiert zur Herstellung eines Arzneimittels für die Behandlung einer bakteriellen Infektion in einem Patienten.

**Patentansprüche für folgenden Vertragsstaat : AT**

1. Ein Verfahren zur Herstellung einer Verbindung der Formel:

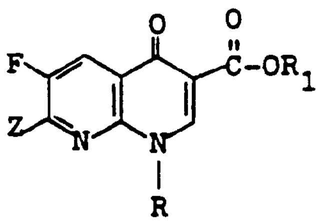

,

worin $R_1$ Wasserstoff oder eine Carboxyschutzgruppe ist; R eine Phenylgruppe der Formel:

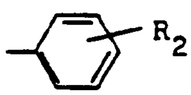

ist, wobei $R_2$ einen oder mehrere Substituenten bedeutet, die unabhängig voneinander aus der Gruppe ausgewählt sind, die aus Wasserstoff, Halogen und Hydroxy besteht; und Z ein heterocyclischer Ring ist, der aus 1-Piperazinyl 1-Pyrrolidinyl und 1-Piperidinyl und substituierten Derivaten davon ausgewählt ist, in welchen der heterocyclische Ring durch einen oder mehrere Substituenten substituiert ist, die unabhängig voneinander aus der Gruppe ausgewählt sind, welche aus $C_1$-$C_6$-Alkyl, Hydroxy, Alkanoyl mit 1 bis 6 Kohlenstoffatomen, Alkanoylamino mit 1 bis 6 Kohlenstoffatomen, und einem Amin der Formel:

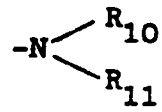

besteht, wobei $R_{10}$ und $R_{11}$ jeweils unabhängig voneinander aus der Gruppe ausgewählt sind, die aus Wasserstoff und $C_1$-$C_6$-Alkyl besteht, mit der Maßgabe, daß, falls Z ein Amino-substituiertes 1-Pyrrolidinyl oder ein unsubstituiertes 1-Piperazinyl bedeutet, R nur eine substituierte Phenylgruppe ist; und pharmazeutisch annehmbarer Salze davon, umfassend

(i) das Umsetzen von ZH mit einer 7-Halogen-1-R-subst.-6-fluor-1,4-dihydro-4-oxo-naphthyridin-3-carbonsäure (A) oder einem Ester davon, worin Z, R und $R_1$ wie oben definiert sind, und X ein Halogen ist,

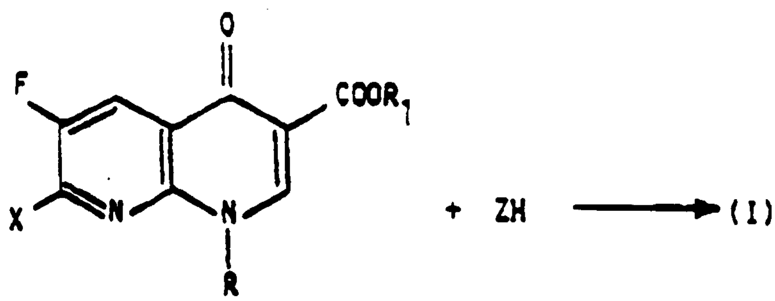

(A)

zur Gewinnung der gewünschten Verbindung (I).

2. Ein Verfahren nach Anspruch 1, welches weiterhin eine oder mehrere der folgenden Vorstufen umfaßt:

(a) das Umsetzen eines 2,6-Dihalogen-5-fluornicotinyl-halogenids mit einem Malonatmonoester zur Gewinnung eines 2,6-Dihalogen-5-fluor-nicotinylacetats (B):

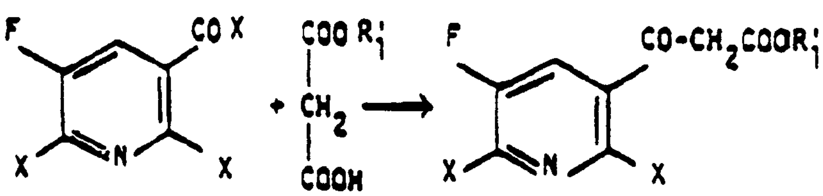

(B) ,

worin $R_1'$ eine Carboxyschutzgruppe ist,

(b) das Umsetzen des β-Ketoesters (B) mit Orthoameisensäuretriethylester und dann mit einem Anilin $RNH_2$ zur Gewinnung eines 3-Anilino-2-(2,6-dihalogen-5-fluornicotinyl)-acrylats (C):

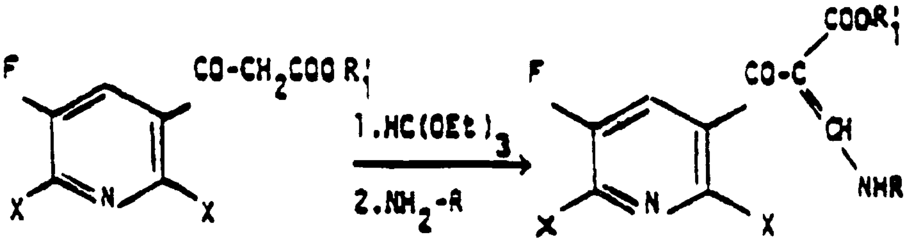


(C) ,

worin R die oben angegebene Bedeutung hat,

(c) das Cyclisieren der Verbindung (C) zur Gewinnung des 1-Phenyl-6-fluor-7-halogen-1,4-dihydro-4-oxo-1,8-naphthyridin-3-carboxylats (A):

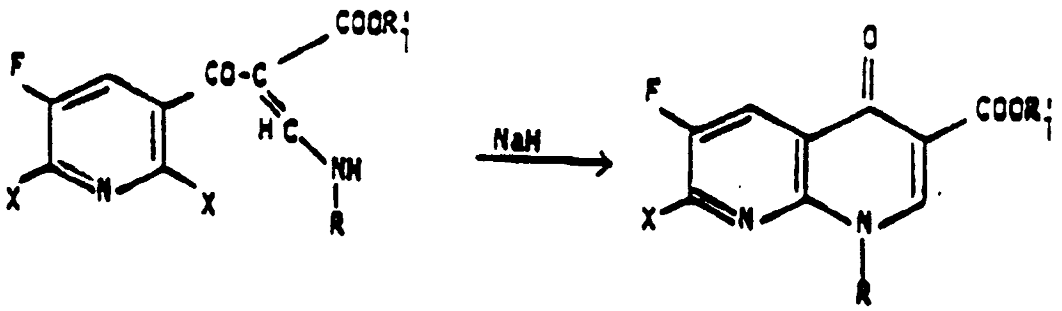


(A)

und

(d) gegebenenfalls das Hydrolysieren von (A) zur Gewinnung der entsprechenden 3-Carbonsäureform davon.

3. Ein Verfahren wie in Anspruch 1 definiert, worin R p-Hydroxyphenyl ist, Z 1-Piperazinyl ist, und $R_1$ Wasserstoff ist.

4. Ein Verfahren wie in Anspruch 1 definiert, worin R p-Fluorphenyl ist, Z 4-Methyl-1-Piperazinyl oder 1-Piperazinyl ist, und $R_1$ Wasserstoff ist.

5. Ein Verfahren wie in Anspruch 1 definiert, worin R p-Fluorphenyl oder o,p-Difluorphenyl ist, $R_1$ Wasserstoff ist, und Z 3-Amino-1-pyrrolidinyl ist.

6. Ein Verfahren zur Herstellung einer antibakterielle Wirksamkeit aufweisenden Zusammensetzung, umfassend das Vermischen einer nach einem Verfahren gemäß Anspruch 1 hergestellten Verbindung mit einem Verdünnungsmittel.

## Revendications

### Revendications pour les Etats contractants suivants : BE, CH, DE, FR, GB, IT, LI, LU, NL, SE

1. Un composé répondant à la formule générale :

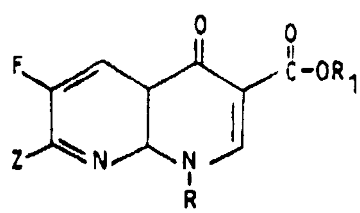

(I)

dans laquelle $R_1$ est un atome d'hydrogène ou un groupe protecteur du groupe carboxy ; R est un groupe phényle de formule :

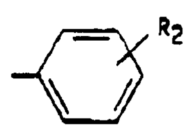

dans laquelle $R_2$ représente un ou plusieurs substituants choisis indépendamment parmi l'hydrogène, les halogènes et les groupes hydroxy ; et Z est un noyau hétérocyclique choisi parmi 1-pipérazinyle, 1-pyrrodinyle et 1-piperidinyle et ses dérivés substitués dans lesquels le noyau hétérocyclique est substitué par un ou plusieurs substituants choisis indépendamment parmi les groupes alkyles en $C_1$-$C_6$, hydroxy, alcanoyles en $C_1$-$C_6$, alcanoylamino en $C_1$-$C_6$, un groupe amino de formule :

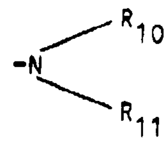

dans laquelle $R_{10}$ et $R_{11}$ sont choisis chacun indépendamment parmi l'hydrogène et les groupes alkyles en $C_1$-$C_6$, avec la condition que si Z est un 1-pyrrolidinyle amino-substitué, ou un 1-pipérazinyle non substitué, le reste R est seulement un groupe phényle substitué ; et ses sels acceptables en pharmacie.

2. Un composé selon la revendication 1, caractérisé en ce que R est p-hydroxyphényle, Z est 1-pipérazinyle et $R_1$ est l'hydrogène.

3. Un composé selon la revendication 1, caractérisé en ce que R est p-fluorophényle, Z est 4-méthylpipérazinyle ou 1-pipérazinyle et $R_1$ est l'hydrogène.

4. Un composé selon la revendication 1, caractérisé en ce que R est un groupe p-fluorophényle ou o,p-difluorophényle, $R_1$ est l'hydrogène et Z est 3-amino-1-pyrrolidinyle.

5. Une composition douée d'activité antibactérienne sous une forme de dosage pharmaceutique contenant un diluant et un composé tel que défini à la revendication 1.

6. Utilisation d'un composé tel que défini à la revendication 1 pour préparer un médicament pour le traitement d'une infection bactérienne chez un patient.

**Revendications pour l'Etat contractant suivant : AT**

1. Un procédé pour fabriquer un composé de formule générale :

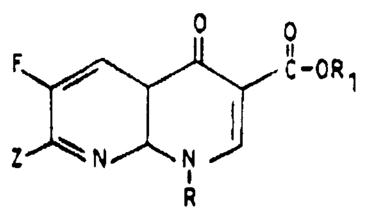

(I)

dans laquelle $R_1$ est un atome d'hydrogène ou un groupe protecteur du groupe carboxy ; R est un groupe phényle de formule :

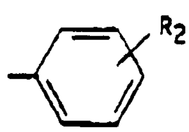

dans laquelle $R_2$ représente un ou plusieurs substituants choisis indépendamment parmi l'hydrogène, les halogènes et les groupes hydroxy ; et Z est un noyau hétérocyclique choisi parmi 1-pipérazinyle, 1-pyrrodinyle et 1-piperidinyle et ses dérivés substitués dans lesquels le noyau hétérocyclique est substitué par un ou plusieurs substituants choisis indépendamment parmi les groupes alkyles en $C_1$-$C_6$, hydroxy, alcanoyles en $C_1$-$C_6$, alcanoylamino en $C_1$-$C_6$, un groupe amino de formule :

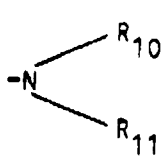

dans laquelle $R_{10}$ et $R_{11}$ sont choisis chacun indépendamment parmi l'hydrogène et les groupes alkyles en $C_1$-$C_6$, avec la condition que si Z est un noyau hétérocyclique amino-substitué 1-pyrrolidinyle ou un 1-pipérazinyle non substitué, le reste R est seulement un groupe phényle substitué ; et ses sels acceptables en pharmacie, caractérisé en ce qu'il consiste

(1) à faire réagir ZH avec un acide 7-halogéno-1-R-6-fluoro-1,4-dihydro-4-oxo-naphtyridine-3-carboxylique (A) ou un de ses esters, dans lequel Z, R et $R_1$ sont définis comme ci-dessus et X est un halogène

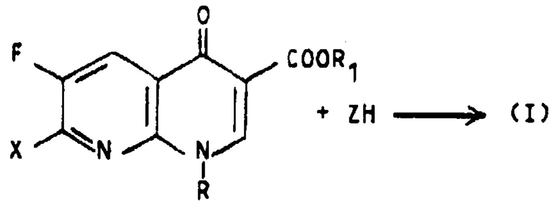

(A)

pour obtenir le composé (I) désiré.

**2.** Un procédé selon la revendication 1, caractérisé en ce qu'il comprend en outre un ou plusieurs des stades opératoires préliminaires suivants :

(a) on fait réagir un halogénure de 2,6-dihalogéno-5-fluoro-nicotinyle avec un monoester malonique pour donner un 2,6-dihalogéno-5-fluoro-nicotinylacétate (B) :

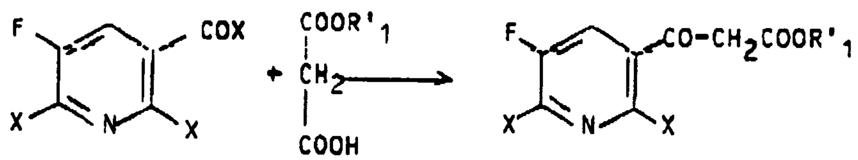

(B)

dans lequel $R'_1$ est un groupement protecteur du groupe carboxy ;

(b) on fait réagir le β-cétoester (B) avec l'orthoformiate de triéthyle et ensuite avec une aniline $RNH_2$ pour donner un 3-anilino-2-(2,6-dihalogéno-5-fluoronicotinyl)-acrylate (C):

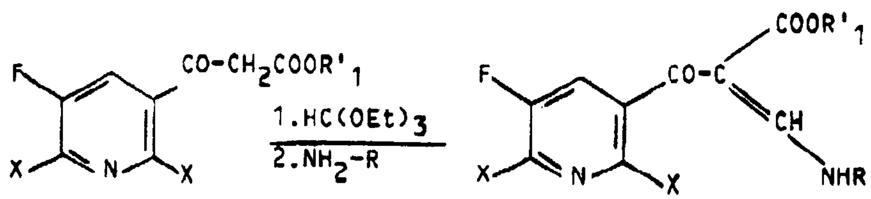


(C)

dans lequel R est tel que défini ci-dessus ;

(c) on cyclise le composé (C) pour obtenir le 1-phényl-6-fluoro-7-halogéno-1,4-dihydro-4-oxo-1,8-naphtyridine-3-carboxylate (A) :

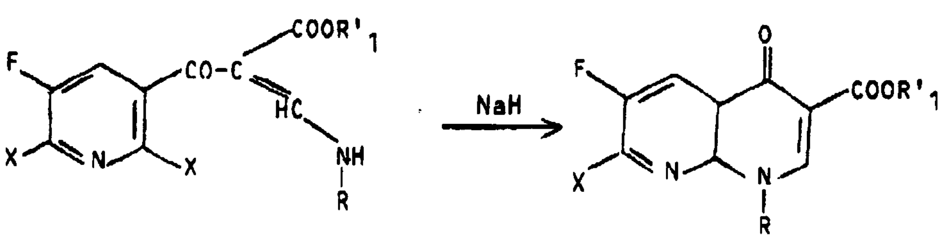


(A)

et

(d) on hydrolyse facultativement (A) pour obtenir sa forme acide 3-carboxylique correspondante.

3. Un procédé selon la revendication 1, caractérisé en ce que R est un groupe p-hydroxyphényle, Z est 1-pipérazinyle et $R_1$ est l'hydrogène.

4. Un procédé selon la revendication 1, caractérisé en ce que R est p-fluorophenyle, Z est 4-méthyl-1-piperazinyle ou 1-piperazinyle et $R_1$ est 1-hydrogène.

5. Un procédé selon la revendication 1, caractérisé en ce que R est un groupe p-fluorophényle ou o,p-difluorophényle, $R_1$ est l'hydrogène et Z est un groupe 3-amino-1-pyrrolidinyle.

6. Un procédé pour préparer une composition douée d'activité antibactérienne, caractérisé en ce que l'on mélange un composé préparé par un procédé selon la revendication 1 avec un diluant.